(19) Europäisches Patentamt

European Patent Office

Office européen des brevets

(11) Numéro de publication : **0 267 213 B1**

(12) **FASCICULE DE BREVET EUROPEEN**

(45) Date de publication du fascicule du brevet :
20.03.91 Bulletin 91/12

(51) Int. Cl.⁵ : **A61M 5/14**

(21) Numéro de dépôt : 87902556.7

(22) Date de dépôt : 24.04.87

(86) Numéro de dépôt international :
PCT/FR87/00138

(87) Numéro de publication internationale :
WO 87/06473 05.11.87 Gazette 87/24

(54) **DISPOSITIF IMPLANTABLE RECHARGEABLE POUR AUTO-INJECTION DOSEE ET REPETEE DE MEDICAMENT.**

(30) Priorité : 24.04.86 FR 8605927

(43) Date de publication de la demande :
18.05.88 Bulletin 88/20

(45) Mention de la délivrance du brevet :
20.03.91 Bulletin 91/12

(84) Etats contractants désignés :
AT BE CH DE FR GB IT LI LU NL SE

(56) Documents cités :
EP-A- 0 138 648
EP-A- 0 143 503
FR-A- 2 569 987
US-A- 4 258 711
US-A- 4 337 770
US-A- 4 544 371
US-A- 4 588 394

(73) Titulaire : **CENTRE NATIONAL DE LA RECHERCHE SCIENTIFIQUE**
**15, quai Anatole France**
**F-75007 Paris (FR)**

(72) Inventeur : **REY, Pierre**
**18, rue Aristide Briand**
**F-77400 Lagny (FR)**
Inventeur : **LEANDRI, Jacqueline**
**50, avenue de Clichy**
**F-75018 Paris (FR)**
Inventeur : **ABBOU, Clément**
**43, avenue de la Dame Blanche**
**F-94120 Fontenay-sous-Bois (FR)**
Inventeur : **SEZEUR, Alain**
**15, rue Raspail**
**F-94230 Cachan (FR)**

(74) Mandataire : **Orès, Bernard et al**
**Cabinet ORES 6, Avenue de Messine**
**F-75008 Paris (FR)**

## Description

La présente invention est relative à un dispositif implantable d'auto-injection répétée de médicament, et notamment d'analgésique, en doses unitaires et à une fréquence qui est fonction des besoins du traitement thérapeutique, et en particulier de l'action analgésique souhaitée.

Actuellement, notamment en ce qui concerne les drogues prescrites dans les traitements anti-douleurs, celles-ci sont administrées à un patient en fonction de l'état de gravité de sa maladie et de l'intensité du syndrome douloureux.

– soit en injections ponctuelles, ce qui pose dans de nombreux cas le problème de piqûres répétées,

– soit dans un site préalablement implanté en position sous-cutanée coopérant avec un cathéter implanté au niveau de la zone d'action analgésique, cette solution ne supprimant néanmoins pas le problème des piqûres répétées.

Il existe plusieurs dispositifs implantables du typye susdit, notamment décrits par les Brevets US-4 588 394, FR-2 569 987 et EP-143 503 – qui toutefois sont conçus pour éviter les interventions involontaires et qui, de ce fait, n'empêchent pas les surdosages par intervention (volontaire) indue, notamment par oubli – ainsi que par les Brevets US-4 193 397 et US-4 258 711, qui décrivent un "pancréas artificiel" destiné à l'administration en continu d'un médicament (dite administration basale) éventuellement renforcée par une administration visant à faire face à des situations exceptionnelles, notamment lors des repas (et correspondant à l'administration d'un "bolus" destiné à s'écouler progressivement vers la zone concernée de l'organisme).

On connaît aussi le Brevet US-4 544 371 qui concerne un dispositif implantable conforme au préambule de la revendication 1 et destiné à l'administration de doses unitaires de médicament par dosage calibré ("metering") de ce dernier. A cet effet, le conduit d'accès à une chambre de pré-dosage est réalisé, au moins à l'intérieur d'un corps élastique délimitant cette chambre, sous la forme d'un capillaire déformable. Toutefois, la réalisation de ce moyen de contrôle rend la construction du dispositif d'injection peu facilement adaptable aux changements de fréquence d'administration de la dose thérapeutique de médicament, en fonction de la nature de celui-ci. En outre, le moyen de soupape de la valve de contrôle de l'injection présente une structure qui est peu satisfaisante notamment lorsqu'il doit être placé au niveau de la zone d'action thérapeutique du médicament, là où est implanté le cathéter

La présente invention a pour but de pourvoir à un dispositif d'injection répétée de doses unitaires de médicament, et notamment d'analgésique, qui répond mieux aux nécessités de la pratique que les dispositifs actuellement connus, notamment en ce que :

– il permet l'injection sans piqûre de la part du patient lui-même, et ce suivant les besoins, en particulier des doses analgésiques strictement nécessaires,

– les piqûres sont nécessaires pour l'approvisionnement en médicament, et notamment en analgésique, et non pas pour l'injection de ce dernier, en sorte que leur nombre est considérablement réduit et deux piqûres consécutives sont très espacées dans le temps, ce qui réduit sensiblement les risques d'infections et, de ce fait, permet d'effectuer par exemple l'injection par voie intrarachidienne et non plus par voie péridurale, ce qui à son tour permet de réduire les doses efficaces d'analgésique d'un facteur au moins égal à 3, et

– il assure la nécessaire sécurité d'emploi en interdisant les surdosages.

La présente invention a pour objet un dispositif d'injection répétée de médicament en doses unitaires et à une fréquence qui est fonction des besoins du traitement thérapeutique, ledit dispositif étant destiné à être implanté dans le corps d'un patient et comportant :

– un site d'injection du médicament, destiné à être implanté au-dessous de la peau du patient ;

– un moyen externe d'alimentation de ce site, comportant notamment une aiguille de piqûre dudit site, relié à une seringue ou à une pompe ;

– un cathéter d'injection de chaque dose unitaire de médicament, destiné à être implanté dans la zone d'action thérapeutique ;

– un réservoir, réalisé en une matière plastique souple, telle qu'un élastomère de silicone, destiné au stockage du médicament entre deux opérations d'approvisionnement à l'aide de ladite seringue et par l'intermédiaire dudit site d'injection ;

– un premier tube de liaison réalisé en matière plastique souple, telle que celle utilisée pour le réservoir, et reliant ce réservoir audit site d'injection ;

– un ballonnet pré-dosé, ayant une entrée et une sortie et destiné à la commande de l'injection de médicament, réalisé lui aussi en matière plastique souple, telle que celle utilisée pour le réservoir, dont le volume est calculé en fonction de la dose unitaire de médicament à injecter par l'intermédiaire dudit cathérer et par pression de ce ballonnet obtenue par déformation digitale cutanée;

– un deuxième tube de liaison entre le ballonnet et le réservoir, lui aussi réalisé comme ce dernier dans une matière plastique souple ;

– une valve équipée d'un moyen de soupape du type sensible uniquement à la pression exercée sur le ballonnet ;

– un moyen de contrôle du débit d'écoulement du médicament, qui est disposé en amont du ballonnet de commande, entre ce dernier et le réservoir et qui est destiné à contrôler le temps de remplissage du ballonnet et donc la fréquence d'injection de la dose unitaire de médicament contenue dans ce dernier ;

lequel dispositif est caractérisé en ce que ledit moyen de soupage est constitué par une tige de soupage enserrée de façon étanche par une gaine qui est réalisée en matière plastique souple présentant un retrait à la polymérisation, à savoir rétrécissable, en ce que cette tige de soupape est pourvue de moyens d'amorce du desserrage de la gaine précitée sous l'effet de la pression de médicament exercée par la commande digitale cutanée du ballonnet précité, et en ce qu'une valve anti-retour est ménagée immédiatement en amont du ballonnet de commande.

Conformément à l'invention, le dispositif se caractérise en outre en ce que le moyen de contrôle du débit d'écoulement est constitué par un tube qui est pourvu d'un micro-canal qui a un diamètre et une longueur définis en fonction du débit souhaité et dont la paroi externe est fixée de façon étanche à la paroi interne du deuxième tube de liaison.

Il est à noter que les tubes (premier et deuxième) sont réalisés en une seule pièce à trois branches, notamment en forme de T ou Y, ce qui permet de choisir la zone d'implantation dudit réservoir dans l'organisme du patient en fonction des besoins, le moyen de contrôle du débit de remplissage étant disposé dans la branche de ladite pièce qui est reliée au ballonnet.

Selon une disposition avantageuse de l'objet de l'invention, l'entrée et la sortie du ballonnet sont ménagées dans le ballonnet de commande en des emplacements situés du même côté.

Outre les dispositions qui précèdent, l'invention comprend encore d'autres dispositions qui ressortiront de la description qui va suivre.

L'invention sera mieux comprise à l'aide du complément de description qui va suivre, qui se réfère aux dessins annexés dans lesquels :

– la figure 1 représente de façon schématique le dispositif selon l'invention, implanté dans un organisme,

– la figure 2 représente à plus grande échelle un détail d'un moyen de soupape utilisé dans le dispositif de la figure 1,

– la figure 3 représente schématiquement une variante dans la disposition relative des composants du dispositif selon l'invention par rapport à la disposition illustrée à la figure 1,

– les figures 4 et 6 correspondent à des variantes de réalisation du dispositif selon l'invention par rapport à la représentation de la figure 3, la figure 4 différant par rapport à la figure 6 en ce que le dispositif comporte un moyen de soupape logé dans l'extrémité distale du cathéter et dont la conception est différente par rapport au moyen de soupape représenté aux figures 1 et 6 (elle est basée sur la Demande de Certificat d'Addition FR-A-2 597 748),

– la figure 5 illustre à plus grande échelle la variante du moyen de soupape utilisé dans le dispositif de la figure 4.

Il doit être bien entendu, toutefois, que ces dessins et les parties descriptives correspondantes sont donnés uniquement à titre d'illustration de l'objet de l'invention, dont ils ne constituent en aucune manière une limitation.

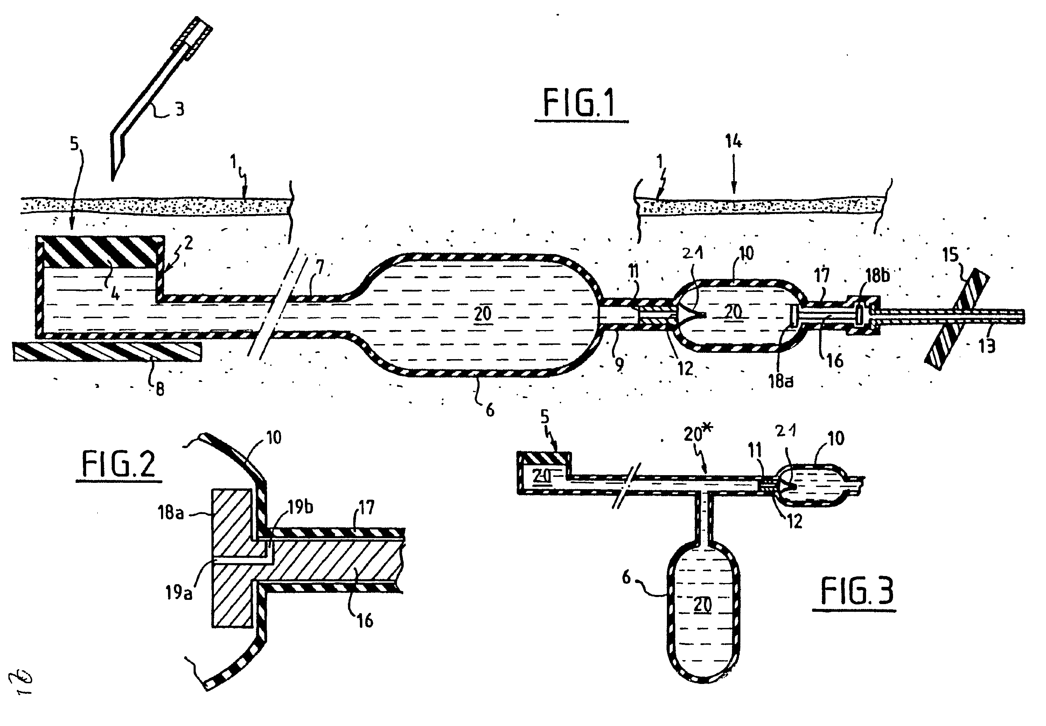

Le dispositif selon l'invention est représenté schématiquement à la figure 1 comme étant implanté dans un organisme (la référence numérique 1 dénote la peau d'un patient).

D'abord, on note la présence d'une cuvette 2 d'alimentation en un produit liquide analgésique 20 à l'aide d'une seringue (non représentée) pourvue d'une aiguille 3 du type dit de HUBERT : à cet effet, la cuvette est pourvue d'un bouchon 4 réalisé en un matériau permettant des piqûres hypodermiques, notamment en élastomère de silicone.

Cette cuvette 2 avec son bouchon 4 sont destinés à être implantés en position sous-cutanée et définissent un site d'injection 5 dont l'emplacement est connu du médecin ou de l'infirmier soignant et, bien entendu, du malade.

Le site d'injection 5 permet d'alimenter un réservoir souple 6 par l'intermédiaire d'un tube de liaison souple 7.

Une plaquette 8 réalisée en tissu de "Dacron" permet de stabiliser l'implantation du site d'injection 5 par rapport aux tissus environnants du malade, pour que la correspondance entre ce site 5 et la zone de la peau 1 destinée à être piquée soit toujours respectée : ainsi on aura toujours la certitude d'alimenter le réservoir 6 lorsqu'on pique la peau 1 du patient pour accéder au site 5 avec l'aiguille 3. L'emploi du tissu de stabilisation 8 répond donc à des mesures de sécurité impératives.

Un autre tube de liaison 9 relie le réservoir 6 à un ballonnet souple 10 de volume prédéfini et d'injection d'une dose d'analgésique, dont la dose thérapeutique est déterminée d'une façon précise par le volume du ballonnet. Le temps de remplissage de ce ballonnet permet de contrôler automatiquement la fréquence d'injection de la dose unitaire d'analgésique contenue dans le ballonnet, interdisant ainsi les surdosages.

Le moyen de contrôle du débit de remplissage du ballonnet 10 est constitué par un tube capillaire 11. Ce capillaire est pourvu d'un micro-canal 12, dont le débit est régulé en choisissant un diamètre et une longueur appropriée, et est logée dans le tube de liaison 9 : le débit de remplissage est donc définitivement déterminé et ce d'une façon précise, par construction.

De cette manière, on peut contrôler le temps de remplissage du ballonnet 10, qui peut être variable et égal par exemple à 2, 4 ou 6 heures, et même plus jusqu'à 24 heures le cas échéant, suivant la fréquence d'injection souhaitée.

Un cathéter 13, qui est implanté dans la zone d'action analgésique, permet d'injecter la dose d'analgésique contenue dans le ballonnet 10 par pression manuelle ou digitale de ce ballonnet effectuée par l'intermédiaire de la peau à l'endroit 14 (connu du patient) où est implanté le ballonnet 10.

Une rondelle 15 de "Dacron" entourant le cathéter 13 permet de stabiliser l'implantation de ce dernier par rapport aux tissus environnants, ce qui est nécessaire pour que l'injection d'analgésique ait lieu toujours dans la zone d'action analgésique et pas dans une autre zone.

Entre le cathéter 13 et le ballonnet 10 est interposé un moyen de soupape permettant l'écoulement de l'analgésique vers ce cathéter en réponse à une pression exercée sur le ballonnet 10.

Avantageusement ce moyen de soupape est du type décrit dans la Demande de Brevet français FR-A-2 551 656 déposée au nom de la Demanderesse en date du 14 Septembre 1983, à savoir qu'il est constitué par une tige de soupape 16 qui, en condition de repos, est enserrée de façon étanche à l'intérieur d'une gaine 17 reliant le ballonnet 10 au cathéter 13 et réalisée en une matière plastique souple présentant un retrait à la polymérisation, laquelle tige comporte à ces extrémités des moyens aptes à empêcher son déplacement axial sous l'effet de la pression exercée sur le ballonnet 10. Ces moyens de stabilisation de la tige 16 peuvent être constitués, conformément aux dispositions décrites dans la Demande de Brevet FR-A-2 551 656 précitée, par des disques 18a et 18b ou par des projections équivalentes également décrites dans cette dernière Demande.

De plus, l'extrémité proximale de la tige de soupape 16 peut être pourvue (conformément à la Demande de Certificat d'Addition FR-A-2 597 748 se rattachant à la Demande de Brevet susdite FR-A-2 551 656 et déposée simultanément avec la présente invention) de moyens d'amorce du desserrage de la gaine 17 de serrage étanche de cette tige 16, sous l'effet de la pression de l'analgésique contenu à l'intérieur du ballonnet de commande 10. Comme le rappelle la figure 2 annexée à cette description, les moyens d'amorce du desserrage sont constitués de façon avantageuse par un canal axial borgne 19a qui est ménagé dans l'extrémité proximale de la tige de soupape 16 et qui communique avec le ballonnet 10 et avec au moins un canal débouchant à la surface de la tige de soupape 16, au niveau de l'extrémité correspondante de la surface interne de la gaine 17, tel que le canal 19b.

Il est évident que la différence essentielle existant dans l'emploi dudit système de soupape (constitué par la coopération d'une tige 16 et d'une gaine 17 de serrage étanche de cette tige), tel qu'appliqué aux dispositifs objets de la Demande de Brevet FR-A-2 551 656 et à certaines dispositions de la Demande de Certificat d'Addition se rattachant à cette dernière, par rapport au dispositif de la présente invention, consiste en ce que dans ce dernier cas il ne s'agit pas de contrôler l'écoulement d'un fluide externe au ballonnet de commande 10, mais plutôt l'écoulement du fluide contenu à l'intérieur de ce ballonnet, à savoir du produit liquide analgésique.

En outre, il va de soi qu'il est possible de disposer le moyen de soupape au niveau de l'extrémité distale du cathéter 13 au lieu de le placer entre ce dernier et le ballonnet de commande 10. Dans ce cas, il est avantageux d'utiliser une tige de soupape sans renflements d'extrémité, telle que celle représentée aux figures 1b de la Demande de Brevet FR-A-2 551 656 et de ladite Demande d'Addition se rattachant à cette dernière, ainsi qu'aux figures 2 et 4 à 13 accompagnant cette Demande de Certificat d'Addition.

A la figure 3, on a représenté schématiquement une variante du dispositif selon l'invention, dans laquelle la disposition relative de ses composants est différente par rapport à celle illustrée à la figure 1. Cette variante comporte un tube 20 à trois branches qui est en forme de T (mais qui pourrait également être en forme de Y) et établissant la nécessaire connexion entre le site d'injection 5, le réservoir 6 et le ballonnet 10.

Cette variante est particulièrement avantageuse parce qu'elle permet de choisir la zone d'implantation du réservoir 6 dans l'organisme du patient, en fonction des besoins.

Il va de soi que dans ce cas, le tube capillaire 11 est disposé dans la branche du tube 20 reliée au ballonnet 10.

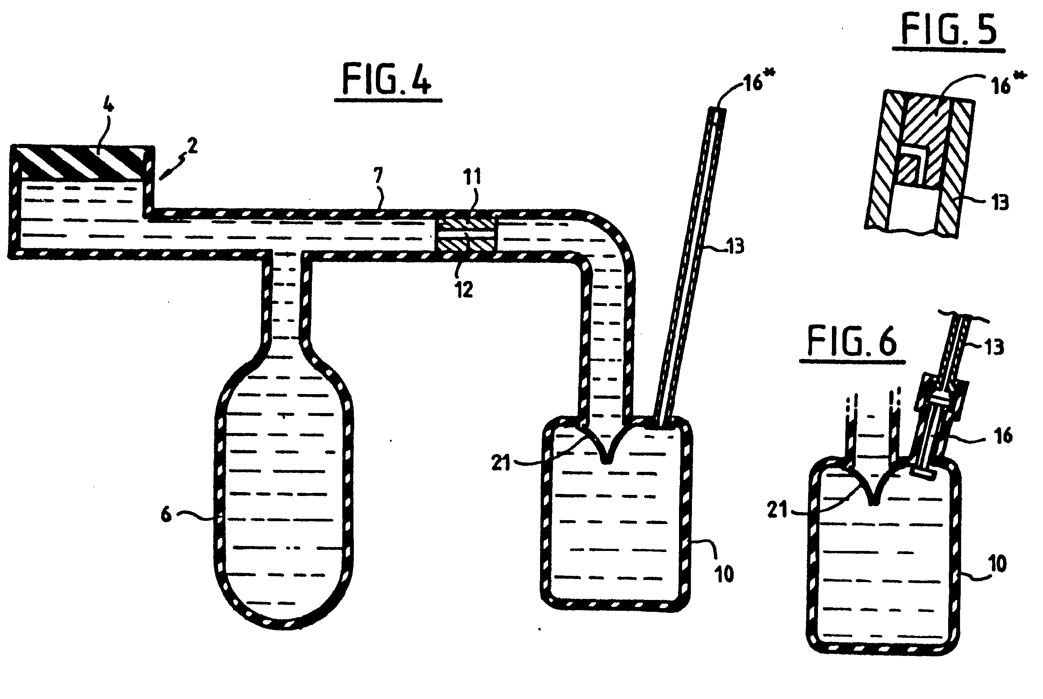

En ce qui concerne les dispositifs représentés aux figures 4 et 6, il y a lieu de noter essentiellement que celles-ci diffèrent du mode de réalisation représenté à la figure 3 en ce que l'entrée et la sortie du ballonnet de commande 10 sont ménagées du même côté de celui-ci. En outre, et ce limitativement à la figure 4, celle-ci diffère (toujours par rapport à la figure 3) en ce que le moyen de soupape 16 de la figure 3, sensible à la pression exercée sur le ballonnet de commande 10, est remplacé par une variante 16*, qui fait l'objet de la Demande de Certificat d'Addition évoquée plus haut aux pages 8 et 9 de cette description (il s'agit de la Demande FR-A-2 597 748 et dont le détail a été représenté à plus grande échelle à la figure 5.

Bien entendu, un moyen de soupape du type représenté à cette figure 5 peut être utilisé non seulement dans le mode de réalisation illustré à la figure 4 (qui est une variante de la figure 6), mais aussi dans le mode de réalisation illustré à la figure 1 en remplaçant la soupape 16 par la soupape 16* précitée.

Dans chaque mode de représentation existe une valve anti-retour (ou anti-reflux) 21, du type dit "à bec de canard" (il s'agit essentiellement d'une valve unidirectionnelle comprenant des lèvres élastiques normalement collabées et réalisées en une seule pièce avec le tube 9), ménagée immédiatement à l'entrée ou, de toutes façons, immédiatement en amont du ballonnet de commande 10. En outre, en ce qui concerne les rapports géométriques entre les différents éléments du dispositif à l'état implanté, tel que représenté aux figures 4 et 6, et la peau du patient, ceux-ci sont sensiblement identiques à ceux définis à la figure 1.

Ci-après est décrit le fonctionnement du dispositif selon l'invention.

Dans un premier temps on remplit de sérum le dispositif qu'on implante ensuite en position sous-cutanée. Le dispositif ainsi implanté est utilisable seulement après 8 à 10 jours, qui est le temps nécessaire pour que le processus de cicatrisation ait lieu.

A l'aide d'une seringue, sur laquelle est montée l'aiguille de HUBERT 3 et qui a un volume supérieur au volume du réservoir 6 (y compris le volume du site d'injection 5 et du tube de liaison 7), on extrait le sérum en piquant la peau malade en correspondance du site 5 et on remplace ce sérum par la drogue à action analgésique, qui est diluée à une concentration déterminée par le médecin, et ce en fonction de l'ation réelle que l'on veut obtenir.

Le volume de drogue diluée, qui peut être stocké dans le réservoir, varie de 20 à 500 cc et même plus, selon les besoins thérapeutiques et est fixé par construction.

En appuyant sur le ballonnet 10 par pression cutanée en correspondance de l'endroit 14, le produit analgésique est injecté, sous l'action de cette pression, dans le cathéter 13, en passant autour de la tige de soupape 16 par dilatation de la gaine 17 qui la serre.

Le relâchement de la pression digitale ou manuelle crée une pression négative, ou dépression, dans le ballonnet 10 (cette dépression est due à la vidange du ballonnet de tout son contenu, laquelle vidante est assurée par le fait que le produit analgésique injecté dans le cathéter ne retourne pas vers le ballonnet de commande de l'injection, étant donné que la gaine 17 se resserre de façon étanche autour de la tige 16).

Or, cette dépression provoque une aspiration de la drogue stockée dans le réservoir 6, dont l'écoulement dans le ballonnet 10 est contrôlé par l'intermédiaire du tube capillaire 11 pourvu du micro-canal 12.

De cette manière les surdosages de drogue sont interdits, car le volume de drogue injecté par pression digitale ne peut pas dépasser le volume de drogue qui passe dans un temps donné, du réservoir 6 vers le ballonnet 10, sous l'action de la différence de pression existant entre eux, ce temps étant défini de façon précise à l'aide du micro-canal 12.

Comme il a été décrit plus haut, le temps de remplissage peut être de 2, 4 ou 6 heures, et même plus, en fonction de la fréquence et de la dose d'injection souhaitées.

Le volume d'analgésique injectable étant déterminé une fois pour toutes, l'effet analgésique est donc essentiellement fonction de la dilution (ou concentration) de drogue dans le réservoir.

Il va de soi que, si l'effet analgésique devait se révéler insuffisant ou si l'on voulait tout simplement le supprimer, il suffirait d'aspirer le produit contenu dans le réservoir au niveau du site 5 et de le remplacer par une drogue plus active (ou plus concentrée) ou par du sérum, respectivement.

En ce qui concerne la quantité de drogue qui retourne vers le réservoir de stockage par le micro-canal, au moment de l'appui sur le ballonnet de commande de l'injection, on peut la considérer comme étant négligeable étant donné la présence du micro-canal et la brièveté du temps d'appui.

Avec le dispositif conforme à l'invention le remplissage du réservoir de stockage ne se fait qu'à la demande et à intervalles éloignés, ce qui diminue d'autant les risques infectieux dûs aux injections quotidiennes. De ce fait, le cathéter d'injection peut être introduit en position intrarachidienne et non plus en position péridurale, ce qui permet de réduire – comme déjà indiqué plus haut – les doses efficaces de drogue d'un facteur au moins égal à 3.

Ainsi que cela ressort de ce qui précède, l'invention ne se limite nullement à ceux de ses modes de réalisation et d'application qui viennent d'être décrits de façon plus explicite ; elle en embrasse, au contraire, toutes les variantes qui peuvent venir à l'esprit du technicien en la matière sans s'écarter du cadre, ni de la portée de la présente invention : en particulier, il va de soi que, bien qu'ayant limité la description à l'auto-injection répétée et contrôlée de doses unitaires d'analgésique, le dispositif selon l'invention s'applique également à l'auto-injection répétée et contrôlée d'un médicament quelconque exerçant une action thérapeutique autre qu'une action analgésique. En particulier, le médicament peut être constitué par de la papavérine ou régitine, qui sont susceptibles de provoquer une érection de la verge par injection dans le corps caverneux de cette dernière.

## Revendications

1. Dispositif d'injection répétée de médicament (20) en doses unitaires et à une fréquence qui est fonction des besoins du traitement thérapeutique, ledit dispositif étant destiné à être implanté dans le corps d'un patient et comportant :

    – un site d'injection (5) du médicament (20), destiné à être implanté au-dessous de la peau (1) du patient ;

– un moyen externe d'alimentation de ce site (5), comportant notamment une aiguille (3) de piqûre dudit site (5), relié à une seringue ou à une pompe ;

– un cathéter (13) d'injection de chaque dose unitaire de médicament, destiné à être implanté dans la zone d'action thérapeutique ;

– un réservoir (6), réalisé en une matière plastique souple, telle qu'un élastomère de silicone, destiné au stockage du médicament (20) entre deux opérations d'approvisionnement à l'aide de ladite seringue et par l'intermédiaire dudit site d'injection (5) ;

– un premier tube de liaison (7) réalisé en matière plastique souple, telle que celle utilisée pour le réservoir, et reliant ce réservoir audit site d'injection (5) ;

– un ballonnet (10) pré-dosé, ayant une entrée et une sortie et destiné à la commande de l'injection de médicament, réalisé lui aussi en matière plastique souple, telle que celle utilisée pour le réservoir (6), dont le volume est calculé en fonction de la dose unitaire de médicament à injecter par l'intermédiaire dudit cathérer (13) et par pression de ce ballonnet (10) obtenue par déformation digitale cutanée ;

– un deuxième tube de liaison (9) entre le ballonnet (10) et le réservoir (6), lui aussi réalisé comme ce dernier dans une matière plastique souple ;

– une valve équipée d'un moyen de soupape (16, 16*) du type sensible uniquement à la pression exercée sur le ballonnet ;

– un moyen de contrôle du débit d'écoulement du médicament, qui est disposé en amont du ballonnet de commande (10), entre ce dernier et le réservoir (6) et qui est destiné à contrôler le temps de remplissage du ballonnet et donc la fréquence d'injection de la dose unitaire de médicament (20) contenue dans ce dernier ;

lequel dispositif est caractérisé en ce que ledit moyen de soupage est constitué par une tige de soupage (16 ; 16*) enserrée de façon étanche par une gaine (17 ; 13) qui est réalisée en matière plastique souple présentant un retrait à la polymérisation, à savoir rétrécissable, en ce que cette tige de soupape est pourvue de moyens d'amorce du desserrage de la gaine précitée (17 ; 13) sous l'effet de la pression de médicament exercée par la commande digitale cutanée du ballonnet précité, et en ce qu'une valve anti-retour (21) est ménagée immédiatement en amont du ballonnet de commande (10).

2. Dispositif selon la revendication 1, caractérisé en ce que ledit moyen de contrôle du débit d'écoulement est constitué par un tube (11) qui est pourvu d'un micro-canal (12) qui a un diamètre et une longueur définis en fonction du débit souhaité et dont la paroi externe est fixée de façon étanche à la paroi interne dudit deuxième tube de liaison (9).

3. Dispositif selon l'une quelconque des revendications 1 ou 2, caractérisé en ce que la gaine enserrant ledit moyen de soupape (16*) est constituée par le cathéter (13), ce moyen de soupape étant disposé à l'extrémité distale du cathéter.

4. Dispositif selon l'une quelconque des revendications 1 ou 2, caractérisé en ce que la gaine enserrant ledit moyen de soupape (16) est constituée par une gaine (17) reliant le ballonnet de commande (10) au cathéter (13), la tige de soupape comportant à ses extrémités des moyens de stabilisation (18a, 18b).

5. Dispositif selon l'une quelconque des revendications 1 à 4, caractérisé en ce que lesdits premier et deuxième tubes sont réunis entre eux de façon à constituer un T ou un Y (20*), ce qui permet de choisir la zone d'implantation dudit réservoir (6) dans l'organisme du patient en fonction des besoins, ledit moyen de contrôle du débit de remplissage étant disposé dans la branche du T ou de l'Y (20*) qui est reliée audit ballonnet (10).

6. Dispositif selon l'une quelconque des revendications 1 à 4, caractérisé en ce que l'entrée et la sortie dudit ballonnet de commande (10) sont ménagées dans ce ballonnet en des emplacements situés du même côté.

## Ansprüche

1. Vorrichtung zum wiederholten Injizieren eines Medikamentes (20) in Einheitsdosen und mit einer Häufigkeit, die von dem Bedarf der therapeutischen Behandlung abhängt, wobei die Vorrichtung zur Implantation in den Körper eines Patienten bestimmt ist und aufweist :

eine Injektionsstelle (5) für das Medikament (20), die zur Implantation unter die Haut (1) des Patienten bestimmt ist,

ein äußeres, mit einer Spritze oder mit einer Pumpe verbundenes Zufuhrmittel für diese Stelle (5) mit insbesondere einer Injektionsnadel (3) für die Stelle (5),

einen Injektionskatheter (13) für jede Einheitsdosis eines Medikamentes, der zur Implantation in den therapeutischen Wirkungsbereich bestimmt ist,

ein aus biegsamem Kunststoff, wie einem Silikonelastomer hergestelltes Reservoir (6), das zum Speichern des Medikamentes (20) zwischen zwei Vorgängen zum Versorgen mit Hilfe der Spritze und über die Injektionsstelle (5) bestimmt ist,

eine erste Verbindungsröhre (7), die aus biegsamem Kunststoff, wie jenem für das Reservoir verwendeten, hergestellt ist und die dieses Reservoir mit der Injektionsstelle (5) verbindet,

einen kleinen vordosierten Beutel (10), der einen Einlaß und einen Auslaß aufweist und zum Steuern des Injizierens des Medikamentes bestimmt ist, wobei auch er aus biegsamem Kunststoff, wie jenem für das Reservoir (6) verwendeten hergestellt ist, dessen Volumen in Abhängigkeit von der Einheitsdosis eines Medikamentes zum Injizieren über den Katheter (13) und durch Drücken dieses kleinen Beutels (10) berechnet ist, das durch eine Deformation der Haut mit einem Finger erhalten wird,
eine zweite Verbindungsröhre (9) zwischen dem kleinen Beutel (10) und dem Reservoir (6), wobei auch sie wie dieses letztere aus einem biegsamen Kunststoff hergestellt ist,
ein Ventil, das mit einem Ventilmittel (16, 16*) des Types versehen ist, der nur auf den auf den kleinen Beutel ausgeübten Druck anspricht,
ein Steuermittel für die Ausflußrate des Medikamentes, welches stromauf des kleinen Steuerbeutels (10) zwischen diesem letzteren und dem Reservoir (6) angeordnet ist und welches zur Steuerung der Füllzeit des kleinen Beutels und somit der Injektionshäufigkeit der Einheitsdosis eines Medikamentes (20) bestimmt ist, die in diesem letzteren enthalten ist, wobei die Vorrichtung dadurch gekennzeichnet ist, daß das Ventilmittel aus einem Ventilstift (16, 16*) besteht, der von einer Hülse (17 ; 13) dichtend umschlossen ist, die aus biegsamem Kunststoff mit einer Polymerisationsschrumpfung im Sinne eines Verengens hergestellt ist, daß dieser Ventilstift mit Mitteln zum Einleiten des Lockerns der Hülse (17 ; 13) unter der Wirkung des durch die Finger-Haut-Steuerung des kleinen Beutels ausgeübten Medikamentendruckes versehen ist, und daß ein Umkehrschutzventil (21) unmittelbar stromauf des kleinen Steuerbeutels (10) angebracht ist.

2. Vorrichtung nach Anspruch 1, dadurch gekennzeichnet, daß das Steuermittel für die Ausflußrate aus einer Röhre (11) besteht, die mit einem Mikrokanal (12) versehen ist, der einen Durchmesser und eine Länge aufweist, die in Abhängigkeit von dem gewünschten Durchsatz definiert sind und dessen Außenwand an der Innenwand der zweiten Verbindungsröhre (9) dichtend befestigt ist.

3. Vorrichtung nach irgendeinem der Ansprüche 1 oder 2, dadurch gekennzeichnet, daß die das Ventilmittel (16*) umschließende Hülse aus dem Katheter (13) besteht, wobei dieses Ventilmittel an dem distalen Ende des Katheters angeordnet ist.

4. Vorrichtung nach irgendeinem der Ansprüche 1 oder 2, dadurch gekennzeichnet, daß die das Ventilmittel (16) umschließende Hülse aus einer Hülse (17) besteht, welche den kleinen Steuerbeutel (10) mit dem Katheter (13) verbindet, wobei der Ventilstift an seinen Enden Stabilisierungsmittel (18a, 18b) aufweist.

5. Vorrichtung nach irgendeinem der Ansprüche 1 bis 4, dadurch gekennzeichnet, daß die ersten und zweiten Röhren miteinander so verbunden sind, daß ein T oder ein Y (20*) gebildet ist, wodurch es ermöglicht ist, den Inplantationsbereich des Reservois (6) in dem Organismus des Patienten abhängig von dem Bedarf zu wählen, wobei das Steuermittel für die Füllrate in dem Zweig des T oder des Y (20*) angeordnet ist, der mit dem kleinen Beutel (10) verbunden ist.

6. Vorrichtung nach irgendeinem der Ansprüche 1 bis 4, dadurch gekennzeichnet, daß der Einlaß und der Auslaß des kleinen Steuerbeutels (10) in diesem kleinen Beutel an Stellen angebracht sind, die auf derselben Seite gelegen sind.

## Claims

1. A device for repeated injection of medication (20) in unit doses at a frequency which is a function of therapy requirements, said device being for implanting in the body of a patient, and comprising :
a medication injection site (5) for implanting beneath the skin (1) of the patient ;
external means for supplying medication to said site (5), the external means comprising, in particular, a needle (3) for puncturing said site (5) and connected to a syringe or a pump ;
a catheter (13) for injecting each unit dose of medication, and for implanting in the therapeutic action zone ;
a supply (6) made of flexible plastics material such as a silicone elastomer, for storing medication (20) between two successive filling operations performed by means of said syringe and via said injection site (5) ;
a first link tube (10) made of flexible plastics material such as that used for the supply, and connecting said supply to said injection site (5) ;
a premetered bulb (10) having an inlet and an outlet and intended for controlling injection of the medication, the bulb being likewise made of flexible plastics material such as that used for the supply (6), and the volume of the bulb being designed as a function of the unit dose of medication to be injected via said catheter (13) by applying pressure on said bulb (10) by deforming the skin with the fingers ;
a second link tube (9) between the bulb (10) and the supply (6), said tube likewise being made like the supply from a flexible plastics material ;
a valve fitted with a valve means (16, 16*) of the type responsive solely to the pressure exerted on the bulb ; and
medication flow rate control means disposed upstream from the control bulb (10) between the bulb and the supply (6) and intended to control the time taken to fill the bulb, thereby controlling the frequency with which a unit dose of medication (20) contained in the bulb is injected ;
said device being characterized in that said valve means is constituted by a valve rod (16 ; 16*) held in liquid-tight manner by a sheath (17 ; 13) which is made of flexible plastics material that shrinks on polymerizing, i.e. a shrinkable material, in that said valve rod is provided with means for initiating release by said sheath (17 ; 13) under the effect of medication pressure exerted by the above-mentioned control of the bulb by applying finger pressure to the skin, and in that a non-return valve (21) is provided immediately upstream from the control bulb (10).

2. A device according to claim 1, characterized in that said flow rate control means is constituted by a tube (11) which is provided with a microchannel (12) having a diameter and a length which are defined as a function of the desired flow rate, and having an outside wall which is fixed in liquid-tight manner to the inside wall of said second link tube (9).

3. A device according to claim 1 or 2, characterized in that the sheath holding said valve means (16*) is constituted by the catheter (13), the valve means being disposed at the distal end of the catheter.

4. A device according to claim 1 or 2, characterized in that the sheath holding said valve means (16) is constituted by a sheath (17) connecting the control bulb (10) to the catheter (13), the valve rod including stabilization means (18a, 18b) at its ends.

5. A device according to any of claims 1 to 4, characterized in that the said first and second tubes are connected together so as to constitute a T-shape or a Y-shape (20*), thereby enabling the zone in which said supply (6) is implanted in the body of the patient to be selected as a function of requirements, said filling flow rate control means being disposed in the branch of the T-shape or the Y-shape (20*) which is connected to said bulb (10).

6. A device according to any one of claims 1 to 4, characterized in that the inlet and the outlet of the control bulb (10) are provided in said bulb at locations which are situated on the same side thereof.

FIG.1

FIG.2

FIG.3

FIG.5

FIG.6

FIG.4